# EUROPEAN PATENT SPECIFICATION

(11) **EP 1 689 498 B1**
(45) Date of publication and mention of the grant of the patent: **14.01.2009**
(21) Application number: 04769766.9
(22) Date of filing: 01.11.2004
(51) Int. Cl.: A63B 23/12

(54) **SYSTEM FOR PERFORMING INDUCED LIMB MOVEMENTS, PARTICULARLY FOR REHABILITATING AND SPORTS PURPOSES**
SYSTEM FÜR DIE DURCHFÜHRUNG VON INDUZIERTEN GLIEDMASSENBEWEGUNGEN, BESONDERS FÜR REHABILITATIONS- UND SPORTLICHE ZWECKE
SYSTEME PERMETTANT D'INDUIRE DES MOUVEMENTS DE MEMBRES, NOTAMMENT DANS UN BUT DE REEDUCATION OU A DES FINS SPORTIVES

(30) Priority: 04.11.2003 IT MI20032126
(43) Date of publication of application: 16.08.2006
(73) Proprietor: Cornacchiari, Renato, 25020 Capriano del Colle (IT); Bonardi, Fiorenzo, 25062 Concesio (IT); Lonati, Francesco, 25087 Villa di Salò (IT)
(72) Inventor: Cornacchiari, Renato, I-25020 Capriano Del Colle (IT)
(74) Representative: Alagem Modiano, Lara S.
(86) International application number: PCT/IB2004/003583
(87) International publication number: WO 2005/042115

(56) References cited:
- WO-A-01/08755
- US-A- 5 241 952
- US-A- 5 429 140
- US-A- 5 791 351

## Description

### Technical field

The present invention relates to a system for performing induced limb movements, particularly for rehabilitating, sports-related and similar purposes. More particularly, the invention relates to a system for the guided execution of limb movements on the part of a patient, particularly for rehabilitating, sports-related and similar purposes.

### Background art

As is known, the neuromuscular system of a person is meant to coordinate all the motor functions that a person can perform under the control of the brain.

In case of traumatic or circulatory or degenerative events, if there has been a partial and/or momentary interruption of the neuromuscular pathways, the coordination of movements, i.e., the pulses that the brain sends to the nerves by means of the neurons in order to make the person perform movements ordered by the brain, may be damaged, and therefore the patient must undergo rehabilitating treatments in order to require the motor control he has lost.

For this purpose, particular rehabilitation techniques are used in which the patient must perform, with the injured limb, a particular movement, which is traced on the surface; i.e., the patient must follow, with his limb, as much as possible, a preset path.

However, these techniques, which allow to rehabilitate muscle and tendon proprioception sensors, i.e., the sensors meant to detect the quality and quantity of musculo-skeletal contraction/elongation, and somesthetic sensors, i.e., the sensors meant to detect the position of the various segments of the limb, are affected by drawbacks.

First of all, physicians or physiotherapists have an objective difficulty in acquiring the data arriving from the patient, i.e., in tracking the progress gradually made by the patient, since the movement that the patient must perform is usually traced on a surface and therefore a trace of the movements that the patient has performed does not remain in each instance and it is not possible to compare the path that the patient should have followed with the injured limb and the path that he has actually followed, so as to constantly monitor the improvement and/or deterioration that the patient undergoes during therapy.

Moreover, the use of predefined paths, traced on the surface, without any external aid, does not allow to perform the type of therapy in which the patient is ordered to follow a path by applying a certain pressure with the limb in following said path.

The pressure is particularly important, since applying it trains not only the somesthetic sensors but also the muscle and tendon proprioception sensors, which detect the quality and quantity of muscle contraction or elongation.

The foregoing applies both to the case of rehabilitation therapies and to the case of the use of the previously cited techniques for sports- and fitness-related purposes in general.

US-A-5 429 140 discloses a system for performing movements against a force that is imposed externally.

US-A-5 791 351 discloses a motion measurement apparatus for displaying and measuring a body movement.

WO 01/08755 discloses a rehabilitation device that indicates to the users what exercises to perform.

US-A-5 242 952 discloses a therepeutic range-of-motion exercise device.

The aim of the present invention is to provide a system for performing induced limb movements, particularly for rehabilitating, sports and similar purposes, which allows the patient to perform a given work, without the mandatory presence of an operator who submits to the patient the various types of path that he must trace with the injured limb or limbs.

Within this aim, an object of the present invention is to provide a system for performing induced limb movements that allows the patient to work uninterruptedly, with a preset progression of the difficulty of the movements.

Another object of the present invention is to provide a system for performing induced limb movements that allows to have a substantially infinite plurality of different work programs available for the patient.

Another object of the present invention is to provide a system for performing induced limb movements that allows the patient to perform work that is repeatable over time and whose outcome can be verified by an operator.

Another object of the present invention is to provide a system for performing induced limb movements that is highly reliable, relatively simple to provide, and at competitive costs.

This aim and these and other objects that will become better apparent hereinafter are achieved by a system for performing induced limb movements, particularly for rehabilitating, sports-related and similar purposes, as defined in claim 1.

Further characteristics and advantages of the invention will become better apparent from the description of preferred but not exclusive embodiments of the system according to the present invention, illustrated by way of non-limiting example in the accompanying drawings, wherein:
Figure 1 is a block diagram of the system according to the present invention;
Figure 2 is a view of the system according to the present invention, applied to a non-flat surface; and
Figure 3 is a side view of the device.

With reference to the figures cited above, the system according to the present invention, generally designated by the reference numeral 1, comprises data processing means, which are conveniently constituted for example by a personal computer 2 provided with display means 3, which allow to display a plurality of paths, which are programmed in the personal computer 2 and which the patient must follow with the injured limb or limbs that he accordingly wishes to rehabilitate or exercise.

Conveniently, the patient is provided with means adapted to reproduce on any surface a path that is displayed on the display means 3 of the personal computer 2. Said means adapted to reproduce said preset path are conveniently constituted by a peripheral unit or terminal 4, which is adapted to communicate with the personal computer 2, for example by means of an infrared port, and is provided with position sensors 5, which allow to send to the personal computer signals that are adapted to identify at each instant the position of the peripheral unit 4 on a given surface 6 on which the patient moves the peripheral unit 4 in order to reproduce a path that is displayed on the display means 3 of the personal computer 2.

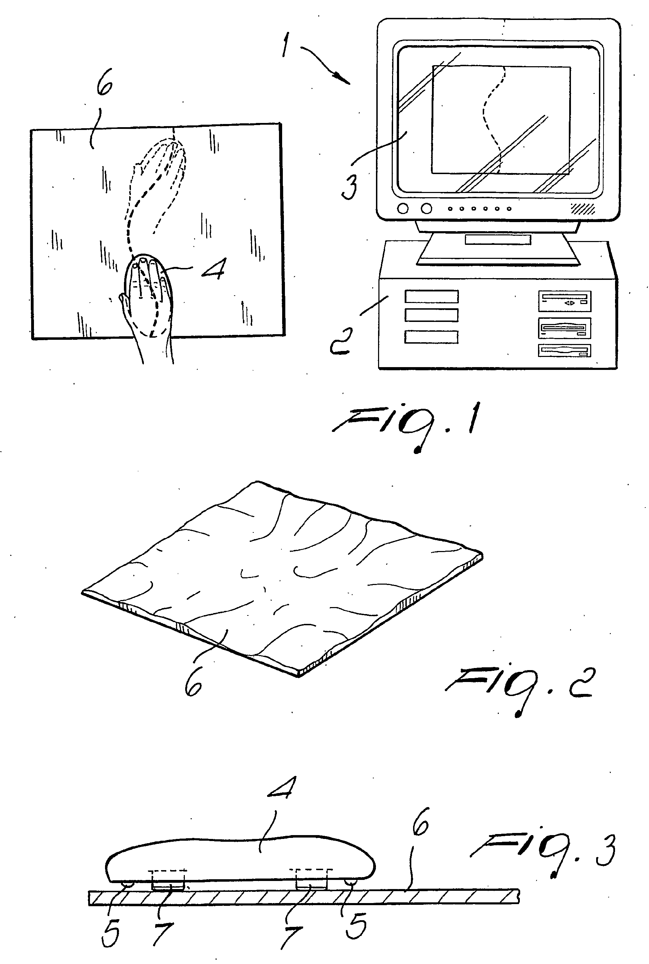

Substantially, if the injured limb is an upper limb, the patient grips the peripheral unit 4, or otherwise, if the injured limb is one of the lower limbs, he rests his foot on the peripheral unit 4, and moves it on the surface 6, which can be either flat or three-dimensional, as shown for example in Figure 2, and tries to produce the path displayed on the display means 3, by appropriately moving the affected limb.

The peripheral unit 4 constantly sends to the personal computer 2 signals that indicate the position of the peripheral unit 4 with respect to the surface 6, so as to display on the display means 3 the path that the patient is actually tracing on the surface 6, in order to be able to then compare it with the predefined path displayed on the display means 3.

The match, or lack thereof, or the offset, between the predefined path that the patient must try to reproduce and the path actually produced by the patient gives the operator an indication of the capabilities and of the health status of the patient in relation to the affected limb.

Conveniently, the personal computer 2 is provided with means that are adapted to process the path traced by the patient and to make a comparison between said path and the predefined path displayed by the display means 3, and to produce a result that indicates the degree of match, or lack thereof, between the two paths.

Advantageously, the peripheral unit 4 can be provided with pressure sensors 7, which are adapted to detect the pressure that the user applies to the peripheral unit 4, as a consequence of a given command issued by the rehabilitation program set on the personal computer 2, and to transmit said pressure data item to the personal computer 2, which is provided with means that are adapted to constantly compare the pressure value read by the pressure sensors 7 with the pressure value that the rehabilitation program expects the patient to apply to the peripheral unit 4.

Conveniently, the connection between the peripheral unit 4 and the processing means constituted by the personal computer 2 may occur not only by means of an infrared port but also by means of any other type of connection, such as cable, wireless and the like.

Conveniently, a plurality of peripheral units 4, for example two, may be provided so that the patient performs a bilateral movement simultaneously for particular rehabilitation requirements.

In practice it has been observed that the system according to the present invention fully achieves the intended aim and objects, since it allows a patient to perform induced limb movements by using peripheral units 4 that are remotely connected to processing means and allow to propose a plurality of different rehabilitation paths, without requiring the presence of an operator in order to modify the path to be submitted to the patient, and especially with the possibility to provide repeatable work that can be verified by the operator, so as to assess the improvements performed in each instance by the patient.

The system thus conceived is susceptible of numerous modifications and variations, all of which are within the scope of the same inventive concept; all the details may further be replaced with other technically equivalent elements.

In practice, the materials used, as well as the contingent shapes and dimensions, may be any according to requirements and to the background art.

## Claims

1. A system (1) for performing induced limb movements, particularly for rehabilitating, sports-related and similar purposes, comprising a central processing unit (2), at least one peripheral unit (4) that is adapted to be used by a patient in order to reproduce a rehabilitation path displayed by said central processing unit (2), said peripheral unit (4) being provided with position sensors (5) that are adapted to transmit position signals to said central processing unit (2) in order to reconstruct the path traced by the patient on said processing unit, **characterized in that** said peripheral unit (4) is moved on a surface (6) to reproduce said rehabilitation path and **in that** the peripheral unit (4) is provided with pressure sensing means (7), which are adapted to send a pressure signal to said processing unit (2), said pressure signal being a measure of the pressure exercised by the patient while reproducing said rehabilitation path.

2. The system according to claim 1, **characterized in that** said processing unit (2) comprises means that are adapted to process said signals that arrive from said sensors (5) of said peripheral unit (4) and to make a comparison between said path traced by the patient and said predefined path displayed by said processing unit (2).

3. The system according to claim 1, **characterized in that** said peripheral unit (4) is connected to said processing unit (2) by means of a radio link.

4. The system according to claim 1, **characterized in that** said at least one peripheral unit (4) is connected to said processing unit (2) by means of an infrared link.

5. The system according to claim 1, **characterized in that** said peripheral unit (4) is connected to said processing unit (2) by means of a cable.

6. The system according to claim 1, **characterized in that** said peripheral unit (4) is moved by the patient over a flat surface (6).

7. The system according to claim 1, **characterized in that** said at least one peripheral unit (4) is moved by the patient over a non-flat surface (6).

8. The system according to claim 7, **characterized in that** said processing unit (2) comprises means that are adapted to process said pressure signal in order to compare it with a pressure value that is preset in said processing unit and is associated with a particular program and path that the patient is following.

## Patentansprüche

1. System (1) zum Ausführen von induzierten Gliedmaßenbewegungen, insbesondere für Rehabilitations-, sportbezogene und ähnliche Zwecke, mit einer zentralen Recheneinheit (2), zumindest einem Peripheriegerät (4), das ausgebildet ist, von einem Patienten verwendet zu werden, um einen Rehabilitationspfad zu reproduzieren, der durch die zentrale Recheneinheit (2) dargestellt wird, das Peripheriegerät (4) ist mit Positionssensoren (5) versehen, die ausgebildet sind, um Positionssignale der zentralen Recheneinheit (2) zu übermitteln, um den durch den Patienten verfolgten Pfad auf der Recheneinheit zu rekonstuieren, **dadurch gekennzeichnet, dass** das Peripheriegerät (4) auf einer Oberfläche (6) bewegt wird, um den Rehabilitationspfad zu reproduzieren und dass das Peripheriegerät (4) mit drucksensierenden Mitteln (7) versehen ist, die ausgebildet sind, um ein Drucksignal an die Recheneinheit (2) zu senden, das Drucksignal ist ein Maß für den Druck, der durch den Patienten ausgeübt wird, während er den Rehabilitationspfad reproduziert.

2. System nach Anspruch 1, **dadurch gekennzeichnet, dass** die Recheneinheit (2) Mittel aufweist, die ausgebildet sind, um die Signale zu verarbeiten, die von den Sensoren (5) des Peripheriegerätes (4) ankommen und einen Vergleich zwischen dem durch den Patienten verfolgten Pfad und dem vordefinierten Pfad, der durch die Recheneinheit (2) dargestellt wird, anzustellen.

3. System nach Anspruch 1, **dadurch gekennzeichnet, dass** das Peripheriegerät (4) mit der Recheneinheit (2) über eine Radioverbindung verbunden ist.

4. System nach Anspruch 1, **dadurch gekennzeichnet, dass** das zumindest eine Peripheriegerät (4) mit der Recheneinheit (2) über eine Infrarotverbindung verbunden ist.

5. System nach Anspruch 1, **dadurch gekennzeichnet, dass** das Peripheriegerät (4) mit der Recheneinheit (2) über ein Kabel verbunden ist.

6. System nach Anspruch 1, **dadurch gekennzeichnet, dass** das Peripheriegerät (4) durch den Patienten über eine ebene Oberfläche (6) bewegt wird.

7. System nach Anspruch 1, **dadurch gekennzeichnet, dass** das zumindest eine Peripheriegerät (4) durch den Patienten über eine nicht ebene Oberfläche (6) bewegt wird.

8. System nach Anspruch 7, **dadurch gekennzeichnet, dass** die Recheneinheit (2) Mittel aufweist, die ausgebildet sind, um das Drucksignal zu verarbeiten, um dieses mit einem Druckwert zu vergleichen, der in der Recheneinheit voreingestellt und mit einem besonderen Programm und dem Pfad, dem der Patient folgt, zugeordnet ist.

## Revendications

1. Système (1) pour exécuter des mouvements de membres induits, en particulier à des fins de rééducation, sportives ou similaires, comprenant une unité centrale (2), au moins une unité périphérique (4) qui est adaptée pour être utilisée par un patient afin de reproduire un trajet de rééducation affiché par ladite unité centrale (2), ladite unité périphérique (4) étant munie de capteurs de position (5) qui sont adaptés pour transmettre des signaux de position à ladite unité centrale (2) afin de reconstruire le trajet tracé par le patient sur ladite unité de traitement, **caractérisé en ce que** ladite unité périphérique (4) est déplacée sur une surface (6) pour reproduire ledit trajet de rééducation et **en ce que** l'unité périphérique (4) est munie de moyens de détection de pression (7), qui sont adaptés pour envoyer un signal de pression à ladite unité de traitement (2), ledit signal de pression étant une mesure de la pression exercée par le patient lors de la reproduction dudit trajet de rééducation.

2. Système selon la revendication 1, **caractérisé en ce que** ladite unité de traitement (2) comprend des moyens qui sont adaptés pour traiter lesdits signaux qui arrivent desdits capteurs (5) de ladite unité périphérique (4) et pour effectuer une comparaison entre ledit trajet tracé par le patient et ledit trajet prédéfini affiché par ladite unité de traitement (2).

3. Système selon la revendication 1, **caractérisé en ce que** ladite unité périphérique (4) est connectée à ladite unité de traitement (2) à l'aide d'une liaison par radio.

4. Système selon la revendication 1, **caractérisé en ce que** ladite unité périphérique au nombre d'au moins une (4) est connectée à ladite unité de traitement (2) à l'aide d'une liaison à infrarouges.

5. Système selon la revendication 1, **caractérisé en ce que** ladite unité périphérique (4) est connectée à ladite unité de traitement (2) à l'aide d'un câble.

6. Système selon la revendication 1, **caractérisé en ce que** ladite unité périphérique (4) est déplacée par le patient sur une surface plane (6).

7. Système selon la revendication 1, **caractérisé en ce que** ladite unité périphérique au nombre d'au moins une (4) est déplacée par le patient sur une surface non plane (6).

8. Système selon la revendication 7, **caractérisé en ce que** ladite unité de traitement (2) comprend des moyens qui sont adaptés pour traiter ledit signal de pression de façon à comparer celui-ci à une valeur de pression qui est pré-établie dans ladite unité de traitement et qui est associée à un programme particulier et à un trajet que le patient est en train de suivre.
